# EUROPEAN PATENT SPECIFICATION

(11) **EP 4 512 307 B1**
(45) Date of publication and mention of the grant of the patent: **19.08.2026**
(21) Application number: 23791576.4
(22) Date of filing: 15.03.2023
(51) Int. Cl.: A61B 3/113, G06F 3/01, G06F 3/0346, G02B 27/01, G06V 40/19, H04N 23/60, H04N 23/611, G02B 27/00, G06F 1/3231, G06F 1/3234, G06F 3/00, G06F 3/04842

(54) **HEAD-MOUNTED INFORMATION PROCESSING DEVICE, INFORMATION PROCESSING METHOD, RECORDING MEDIUM**
KOPFMONTIERTE INFORMATIONSVERARBEITUNGSVORRICHTUNG, INFORMATIONSVERARBEITUNGSVERFAHREN, AUFZEICHNUNGSMEDIUM
DISPOSITIF DE TRAITEMENT D'INFORMATIONS MONTÉ SUR LA TÊTE, PROCÉDÉ DE TRAITEMENT D'INFORMATIONS, SUPPORT D'ENREGISTREMENT

(30) Priority: 22.04.2022 JP 2022070773
(43) Date of publication of application: 26.02.2025
(73) Proprietor: Sony Group Corporation, Tokyo 108-0075 (JP)
(72) Inventor: YAMAZAKI, Sayaka, Tokyo 108-0075 (JP); YAMANO, Ikuo, Tokyo 108-0075 (JP); YOKOYAMA, Masayuki, Tokyo 108-0075 (JP)
(74) Representative: D Young & Co LLP
(86) International application number: PCT/JP2023/010025
(87) International publication number: WO 2023/203923

(56) References cited:
- WO-A1-2021/112990
- JP-A- 2019 528 510
- JP-A- 2021 170 384
- US-A1- 2014 078 283

## Description

### [Technical Field]

The present technology relates to a technical field of a head mount type information processing device, an information processing method, and a storage medium that can acquire various pieces of information on an equipment user.

### [Background Art]

Information processing devices such as head mount displays and smart glasses that are equipped to users' heads can perform more suitable processing by obtaining various pieces of information on equipment users.
For example, following PTL 1 discloses a technique for suitably estimating a user's visual line by obtaining geometric information on an equipment user.

### [Citation List]

### [Patent Literature]

[PTL 1] WO 2016/157486

Previously proposed arrangements are disclosed in US 2014/078283 A1.

### [Summary]

### [Technical Problem]

By the way, various pieces of information that are expected to be acquired from the equipment user are conceived in addition to visual line information. Examples of the various pieces of information include information on a facial expression, information on irises, information on a heart rate, or the like. When these various pieces of information on the equipment user can be acquired, it is possible to provide various experiences to the equipment user.

When various pieces of information on the equipment user are appropriately acquired, it is preferable to appropriately set imaging conditions (imaging parameters) of cameras or light sources for capturing images. However, it is assumed that the imaging conditions differ depending on information that needs to be acquired.

With such a problem in view, it is an object of the present technology to appropriately obtain a plurality of pieces of information on an equipment user.

### [Solution to Problem]

A head mount type information processing device according to the present technology includes: a determination processing unit that determines acquisition target information according to a scene; a setting processing unit that sets an imaging parameter for acquiring an image that is an image of eyes of an equipment user and corresponds to the acquisition target information; and an imaging control unit that captures the image of the eyes of the equipment user according to the imaging parameter.
The acquisition target information differs depending on a use scene of an HMD 1 that is the head mount type information processing device, and is, for example, iris information, visual line information, facial expression information, or heart rate information.
Suitable photographing conditions (imaging parameters) for obtaining these pieces of acquisition target information are different. Examples of the photographing conditions include a position and an angle of view of a camera, arrangement, a light amount, and a wavelength range of use of light sources, or the like.
The acquisition target information is determined according to a scene, so that it is possible to appropriately set the imaging parameters.

### [Brief Description of Drawing]

[Fig. 1]

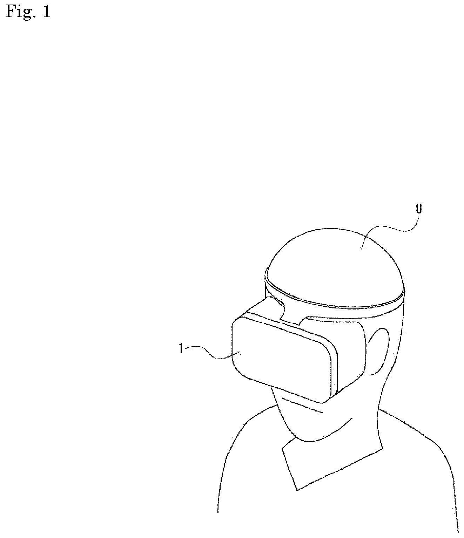

   Fig. 1 is a schematic view illustrating a state where an equipment user is equipped with an HMD.
[Fig. 2]

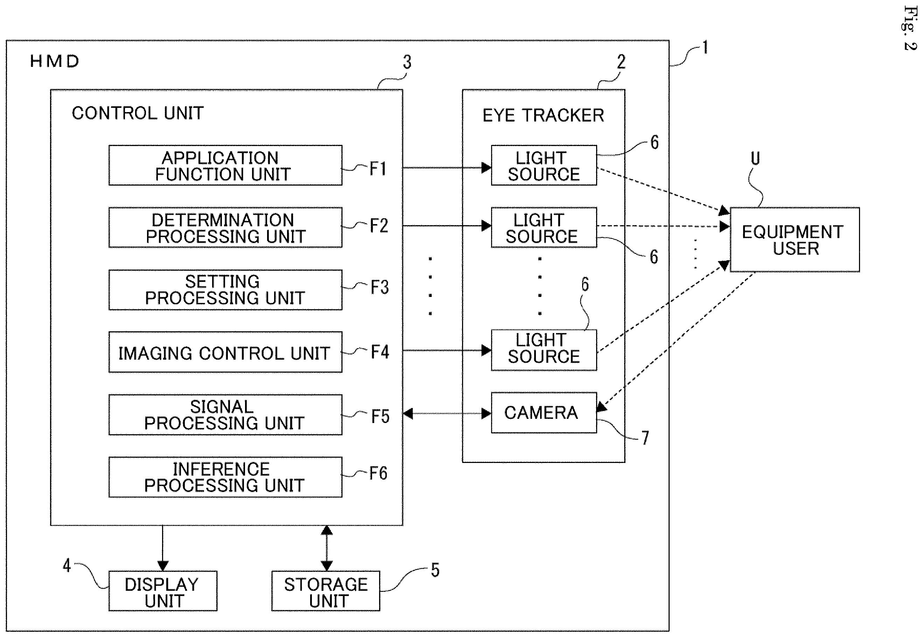

   Fig. 2 is a functional block diagram of the HMD according to a first embodiment.
[Fig. 3]

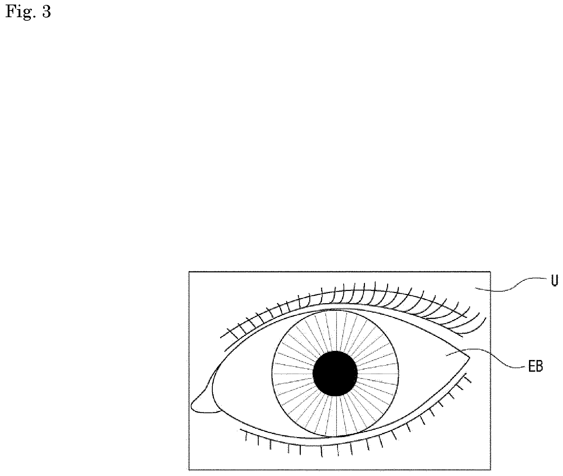

   Fig. 3 is a diagram illustrating an example of an eyeball image.
[Fig. 4]

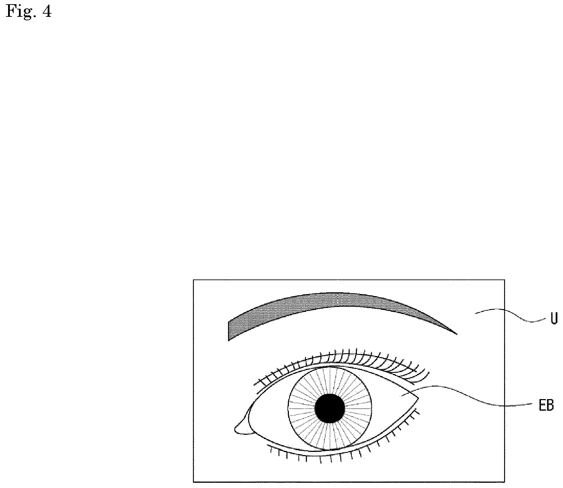

   Fig. 4 is a diagram illustrating an example of a wide angle image.
[Fig. 5]

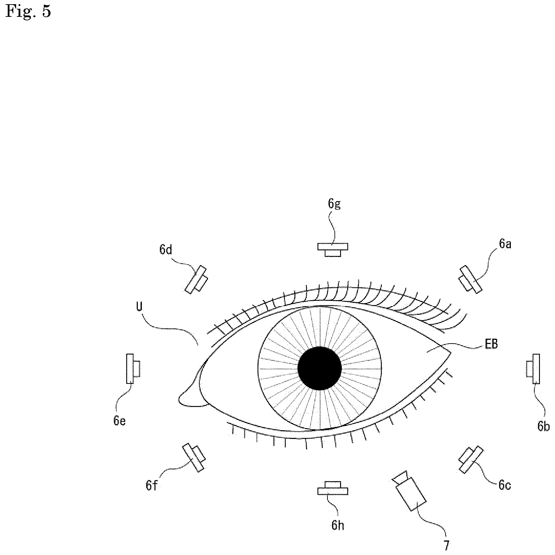

   Fig. 5 is an explanatory view of light sources disposed around an eye of the equipment user.
[Fig. 6]

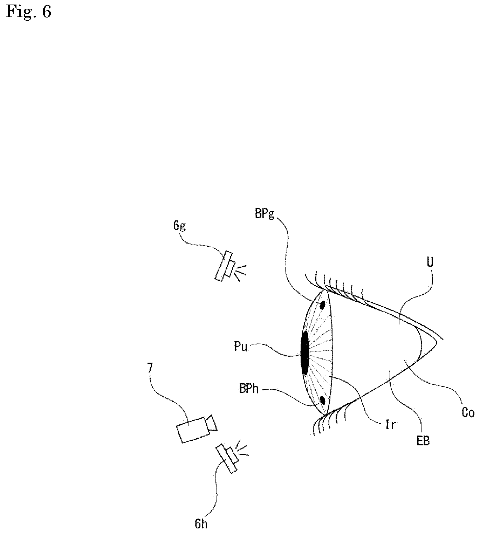

   Fig. 6 is a diagram for explaining a control mode of light sources 6 and a camera 7 based on a first imaging parameter.
[Fig. 7]

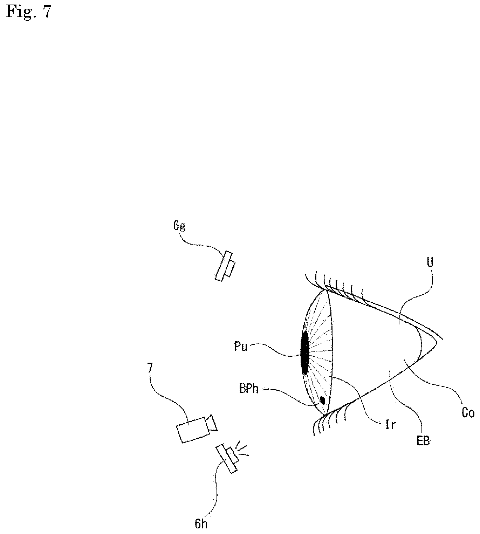

   Fig. 7 is a diagram for explaining a control mode of the light sources 6 and the camera 7 based on a second imaging parameter.
[Fig. 8]

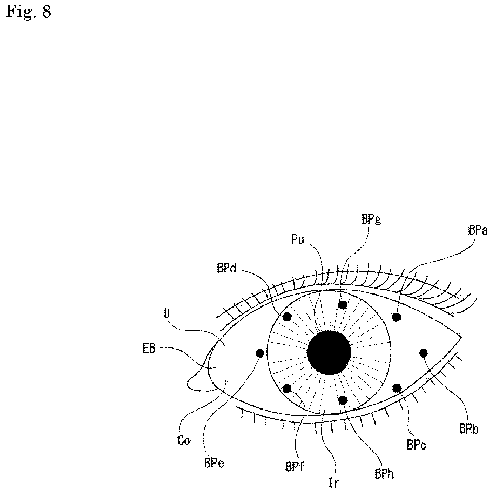

   Fig. 8 is a diagram illustrating bright spots at a time when all light sources are turned on to obtain visual line information.
[Fig. 9]

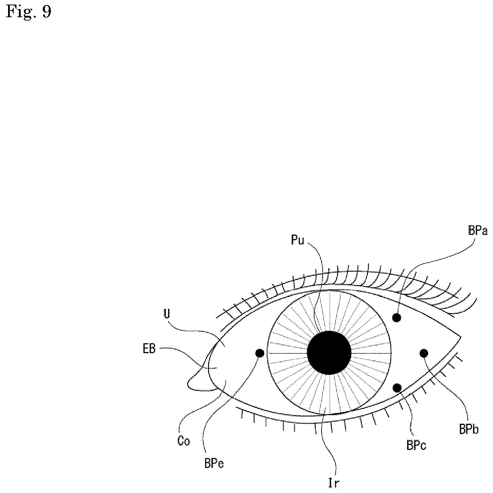

   Fig. 9 is a diagram illustrating bright spots at a time when part of the light sources are turned on to obtain iris information.
[Fig. 10]

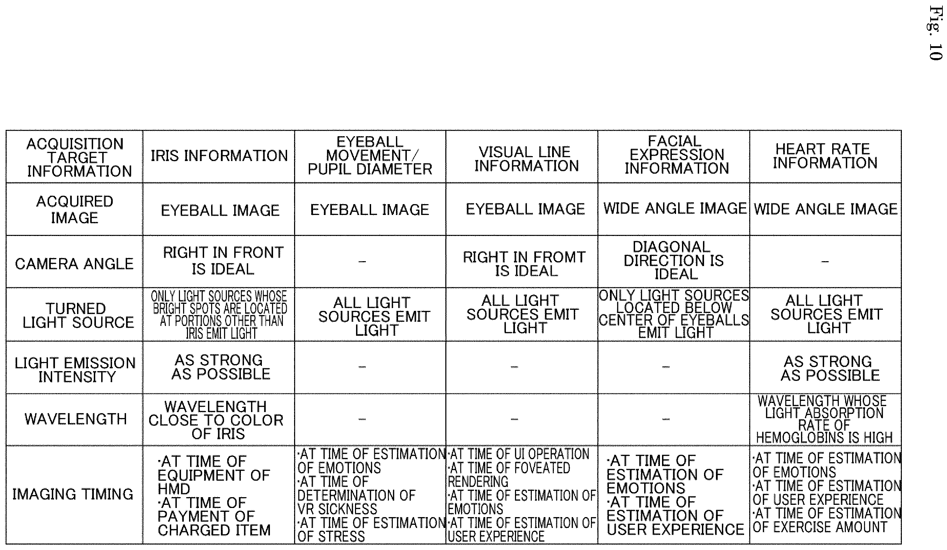

   Fig. 10 illustrates a table illustrating a relationship between acquired information on the equipment user and imaging parameters.
[Fig. 11]

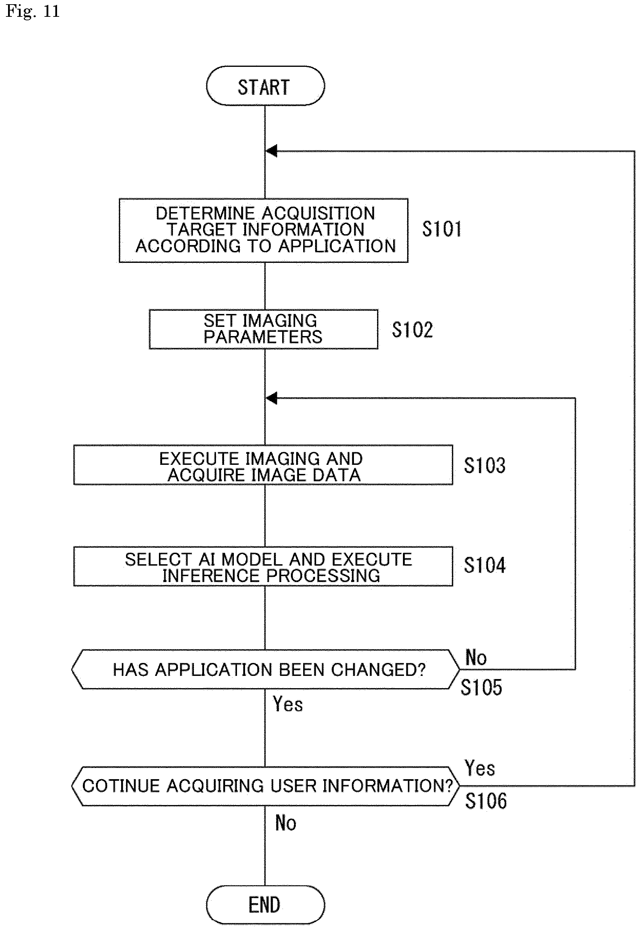

   Fig. 11 is a flowchart illustrating an example of processing executed by a control unit of the HMD.
[Fig. 12]

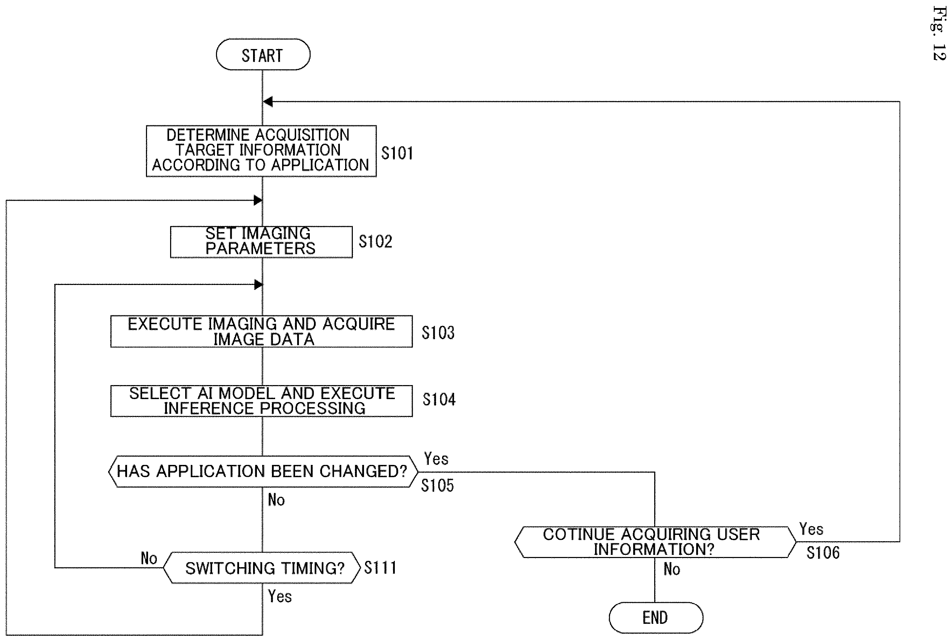

   Fig. 12 is a flowchart illustrating another example of processing executed by the control unit of the HMD.
[Fig. 13]

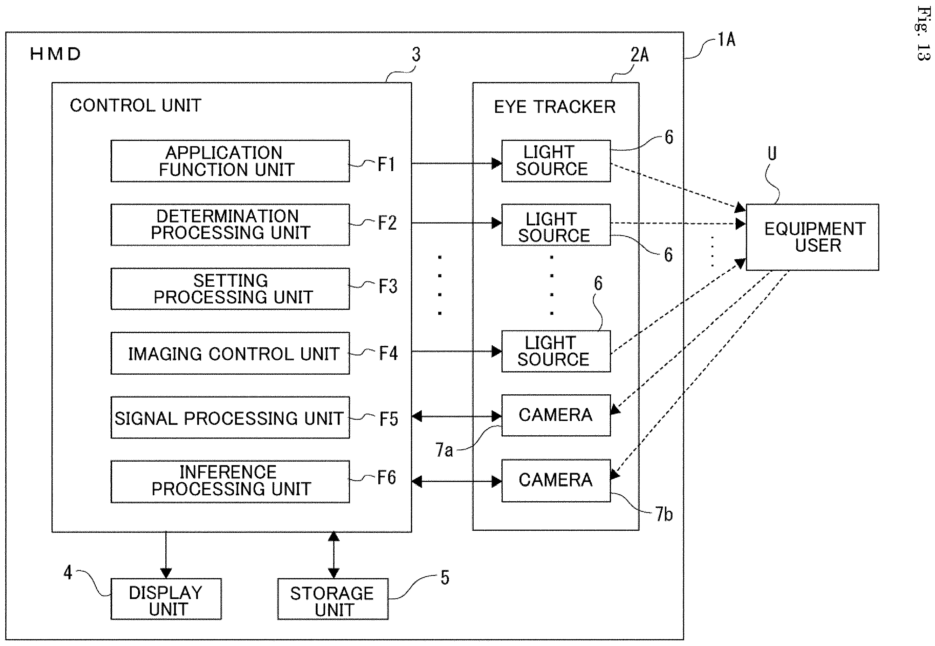

   Fig. 13 is a functional block diagram of the HMD according to a second embodiment.
[Fig. 14]

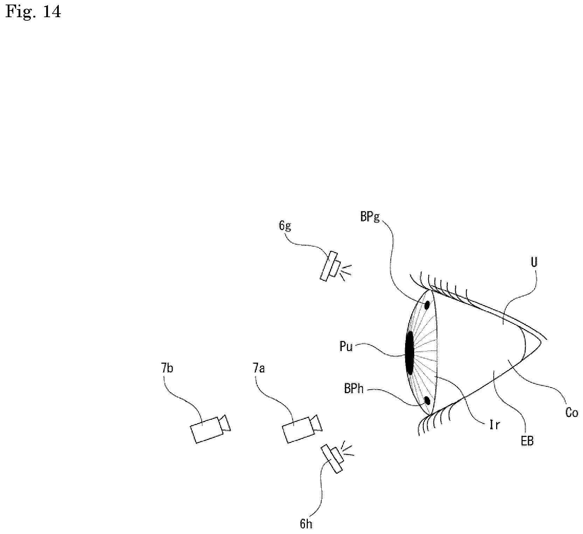

   Fig. 14 is a diagram schematically illustrating an arrangement example of two cameras.
[Fig. 15]

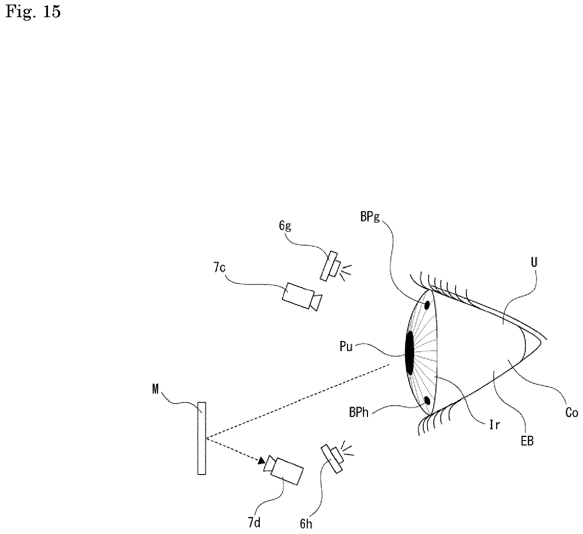

   Fig. 15 is a diagram schematically illustrating an arrangement example in a case where the two cameras and a mirror are used.
[Fig. 16]

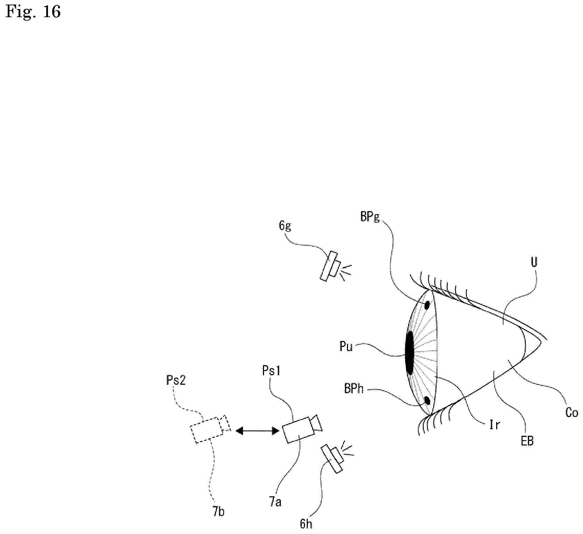

   Fig. 16 is a diagram schematically illustrating how a camera is moved to substitute the two cameras with the one camera.
[Fig. 17]

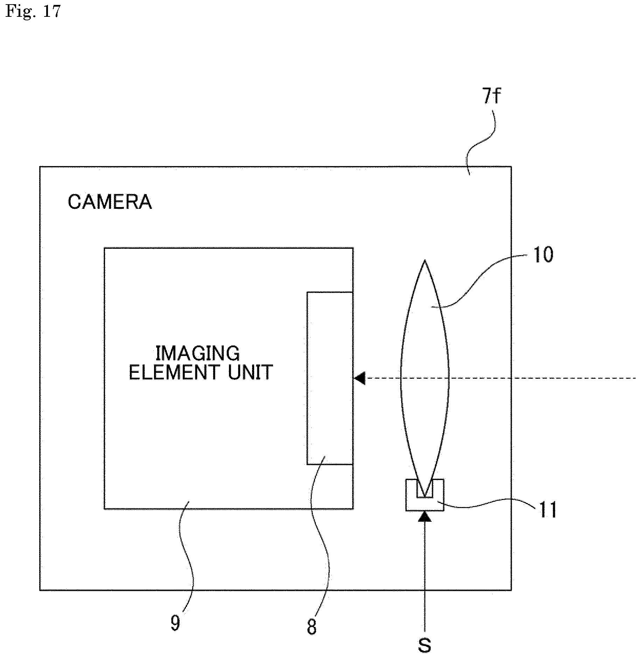

   Fig. 17 is a diagram schematically illustrating a camera including an optical lens that is movable.

### [Description of Embodiment]

Hereinafter, embodiments according to the present technology will be described in the following order with reference to the accompanying drawings.
<1. Aspect of Information Processing Device>
<2. Relationship between Acquired Information and Imaging Parameters>
<3. Processing Flow>
<4. Second Embodiment>
<5. Modification Example>
<6. Summary>
<7. Present Technology>
<1. Aspect of Information Processing Device>

A configuration of the information processing device according to a first embodiment of the present technology will be described.

Note that the information processing device is an information processing device of a type that is equipped to a user's head, and various aspects such as a Head Mount Display (HMD) and a smart glass are conceived.

Although an HMD 1 that is an example of the information processing device and an equipment user U (see Fig. 1) who is equipped with the HMD 1 will be cited and described below, carrying out the present technology is not limited to the form of the head mount display.

The HMD 1 includes an eye tracker 2, a control unit 3, a display unit 4, and a storage unit 5 (see Fig. 2).

The eye tracker 2 employs a configuration for acquiring images of the eyes of the equipment user U of the HMD 1. More specifically, the eye tracker 2 may include one or a plurality of light sources 6 and a plurality of cameras 7.

The images of the eyes include both of an image (see Fig. 3) whose angle of view has been adjusted such that an eyeball EB is largely reflected to acquire a high-resolution image of the eyeball EB of the equipment user U, and an image (see Fig. 4) whose angle of view has been adjusted to capture an image including wrinkles, eyebrows, and the like around the eyes, too.

In the following description, an image obtained by largely imaging the eyeball EB as illustrated in Fig. 3 is described as an "eyeball image", and an image obtained by imaging surroundings of the eyes as illustrated in Fig. 4, too, is described as a "wide angle image".

The eye tracker 2 according to the first embodiment includes the plurality of light sources 6 and one camera.

As illustrated in Fig. 5, the light sources 6 are disposed as light sources 6a, 6b, 6c, 6d, 6e, 6f, 6g, and 6h so as to surround the eye of the equipment user U. By turning on the plurality of these light sources 6, it is possible to capture good eyeball images and wide angle images.

The light source 6 can emit light of a specific wavelength. For example, the light source 6 may be able to emit visible light of a specific wavelength or may be able to emit Infrared (IR) light.

The camera 7 is disposed around the eye in a state where the optical axis is directed to the eyeball EB.

The camera 7 employs a configuration including optical members such as a lens and an iris mechanism that are necessary for imaging, a light reception element that receives incident light incident through the optical members, a read-out circuit that reads charges obtained by photoelectric conversion in the light reception element, and the like.

Note that the camera 7 may include a processing unit that performs Correlated Double Sampling (CDS) processing, Automatic Gain Control (AGC) processing, A/D conversion processing, and the like.

An image signal that is a digital signal is output from the camera 7 to the control unit 3.

Note that, as the light reception element included in the camera 7, various light reception elements such as a light reception element that has sensitivity for Red (R), Green (G), and Blue (B), and a light reception element that has sensitivity for IR light are assumed.
Furthermore, a Time of Flight (ToF) camera may be adopted as the camera 7 to acquire information on a facial expression described later.

The control unit 3 performs various types of processing for integrally controlling the HMD 1. More specifically, the various types of processing include processing of causing the display unit 4 to display images, and the like.

Furthermore, the control unit 3 performs processing for obtaining various pieces of information on the equipment user U.
More specifically, the control unit 3 functions as an application function unit F1, a determination processing unit F2, a setting processing unit F3, an imaging control unit F4, a signal processing unit F5, and an inference processing unit F6 (see Fig. 2).

The application function unit F1 performs processing for implementing functions of various types of applications. Examples of the various types of applications include a user authentication application, a game application, a Cross Reality (XR) application, and an exercise application.

The application function unit F1 that implements the function of the user authentication application performs processing of, for example, acquiring features of the equipment user U of the HMD 1, determining whether or not the equipment user U matches with a registered user, and determining whether or not the equipment user U can be authenticated.

The application function unit F1 that implements the function of the game application and the application function unit F1 that implements the function of the XR application perform processing of causing the equipment user U to visually recognize, as an XR space, a Virtual Reality (VR) space, an Augmented Reality (AR) space, a Mixed Reality (MR) space, or a Substitutional Reality (SR) space via, for example, the display unit 4, processing of making an avatar that is a clone of the equipment user U appear in the space, processing of causing the avatar to reflect the state of the equipment user U, processing of presenting menu items or options to the equipment user U, processing of accepting an operation of the equipment user U and reflecting an acceptance result in display of the display unit 4, communication processing of performing communication with other users via avatars of the other users that appear in the XR space, and the like.

The application function unit F1 that implements the function of the exercise application (or a health care application or a fitness application) performs processing of calculating and presenting an exercise strength according to the motion of the equipment user U, processing of calculating and presenting calorie consumption, processing of presenting an exercise history so far, and the like.

These various types of applications use information on the equipment user U to perform appropriate processing according to the equipment user U.

The determination processing unit F2 performs processing of determining information to be acquired from the equipment user U per application executed by the application function unit F1. Note that it can be also regarded that the determination processing unit F2 determines the information to be acquired from the equipment user U per scene.

In a case where, for example, the application function unit F1 implements the function of the user authentication application, the determination processing unit F2 determines to acquire iris information on the equipment user U.

Furthermore, in a case where the application function unit F1 implements the function of the game application, the determination processing unit F2 determines to acquire visual line information, eyeball movement information, pupil diameter information, heart rate information, and the like on the equipment user U.

Furthermore, in a case where the application function unit F1 implements a function of the Cross Reality (XR) application, the determination processing unit F2 determines to acquire the eyeball movement information, the pupil diameter information, the facial expression information, and the like on the equipment user U.

Furthermore, in a case where the application function unit F1 implements the function of the exercise application, the determination processing unit F2 determines to acquire the heart rate information, respiratory rate information, and the like on the equipment user U.

The setting processing unit F3 sets imaging parameters according to acquisition target information determined by the determination processing unit F2.

Here, the imaging parameters indicate optimal imaging settings for obtaining acquired information determined for the equipment user U, more specifically, indicates settings of the light sources 6 and settings of the camera 7.

Examples of the settings of the light source 6 include settings of whether or not each light source 6 emits light, the position and the orientation of the light source 6, and the intensity and the wavelength range of light to emit, and the like. Furthermore, the settings of the camera 7 are settings of the camera 7 to use, the position, the orientation, and the angle of view of the camera 7, and the like.

A plurality of imaging parameters may be prepared, and appropriately switched according to the acquisition target information. In the present embodiment, the setting processing unit F3 can appropriately switch between a first imaging parameter and a second imaging parameter.

Fig. 6 illustrates an aspect example of the light sources 6 and the camera 7 based on the first imaging parameter. Note that Fig 6 and Fig. 7 described later schematically illustrate only part of the plurality of light sources 6 disposed around the eye of the equipment user U.

The first imaging parameter is settings for turning on both of the light source 6g that is disposed diagonally above the eyeball EB of the equipment user U and the light source 6h that is disposed diagonally below the eyeball EB, and is an imaging parameter that is used for a purpose of improving brightness at a time of photographing, and obtaining a clear image of the eyeball EB.
The first imaging parameter is, for example, a parameter used to obtain the visual line information on the equipment user U.

Fig. 7 illustrates a specific example of the second imaging parameter. The second imaging parameter is settings for turning off the light source 6g that is disposed diagonally above the eyeball EB of the equipment user U, and turning on the light source 6h that is disposed diagonally below the eyeball EB, and an imaging parameter used for a purpose of obtaining an image clearly showing wrinkles (ups and downs of the skin) around the eye. The second imaging parameter is, for example, a parameter used to obtain the facial expression information on the equipment user U.

Note that the bright spots BP illustrated in Figs. 6 and 7 indicate light spots reflected in the eyeball EB due to the light sources 6. More specifically, a bright spot BPg indicates a light spot reflected in the eyeball EB due to the light source 6g, and a bright spot BPh indicates a light spot reflected in the eyeball EB due to the light source 6h.

The bright spot BP may be used as appropriate according to the acquisition target information on the equipment user U.
To obtain, for example, the visual line information on the equipment user U, the light sources 6 are turned on to reflect the bright spots BP on a conjunctiva Co and an iris Ir as illustrated in Fig. 8. The visual line information can be estimated according to a positional relationship between the bright spots BP, the iris Ir, and a pupil Pu.

Furthermore, iris information for performing iris authentication is preferably obtained by capturing an image such that the bright spots BP are not reflected in the iris Ir.

More specifically, when the iris information on the equipment user U is obtained, only the light sources 6a, 6b, 6c, and 6e are turned on, and the light sources 6d, 6f, 6g, and 6h whose bright spots BP are reflected on the iris Ir are turned off as illustrated in Fig. 9. Consequently, it is possible to suitably obtain the iris information.

In addition, the setting processing unit F3 sets the imaging parameters that cause only the light sources 6 such as the light sources 6c, 6f, and 6h located below the center of the eyeball EB to emit light to obtain the facial expression information on the equipment user U, and sets the imaging parameters of the camera 7 such that the angle of view includes up to the surroundings of the eye to obtain the heart rate information.

Fig. 2 is referred back to for description.

The imaging control unit F4 controls a light emitting operation of the light sources 6 and an imaging operation of the camera 7 according to the imaging parameters set by the setting processing unit F3.

More specifically, the imaging control unit F4 performs light emission control on the light sources 6 based on the light source clock signal. Furthermore, the imaging control unit F4 performs imaging control on the camera 7 based on the camera clock signal.
Note that, by synchronizing the light source clock signal and the camera clock signal, the imaging control unit F4 may switch the imaging parameters every time one frame is imaged, and perform imaging. Consequently, it is possible to obtain a different image per frame, so that it is possible to obtain different information on the equipment user U per frame.

Furthermore, the imaging parameters may be switched every other certain frames instead of switching the imaging parameters per frame.

For example, imaging of nine frames of 10 frames may be performed using the imaging parameters for obtaining the visual line information on the equipment user U, and imaging of one remaining frame may be performed using the imaging parameters for obtaining the heart rate information on the equipment user U.

The signal processing unit F5 performs various types of signal processing on the digital signal output from the camera 7. For example, the signal processing unit F5 performs preprocessing, synchronization processing, YC generation processing, resolution conversion processing, and the like as a camera process.

Furthermore, the signal processing unit F5 generates a file for recording or communications by performing, for example, compression coding for recording or communication, formatting, and generation or addition of metadata for, for example, image data having been subjected to the above various types of processing.
The signal processing unit F5 generates an image file in a format such as Joint Photographic Experts Group (JPEG), Tagged Image File Format (TIFF), or Graphics Interchange Format (GIF) as a still image file. Alternatively, an image file may be also generated in an MP4 format or the like that is used for recording moving images and sounds in conformity with MPEG-4. Moreover, an image file may be also generated as RAW image data.

Note that the signal processing unit F5 performs processing of converting the above-described image data into a format of an input image input to an Artificial Intelligence (AI) model used for the inference processing unit F6.

The inference processing unit F6 inputs to the AI model the image data converted by the signal processing unit F5, and obtains an inference result thereof. For example, output data from the AI model is the visual line information, the facial expression information, or the like on the equipment user U.
Note that the inference processing unit F6 may not necessarily need to perform inference processing depending on an application implemented by the application function unit F1.

The inference result of the inference processing unit F6 is used by, for example, the application function unit F1.

When, for example, implementing the function of the user authentication application, the application function unit F1 performs display processing or the like for making the equipment user U visually recognize a notification dialog for notifying according to the inference result the equipment user U of whether or not the equipment user U can be authenticated.

Furthermore, when implementing the function of the game application, for example, the application function unit F1 determines a timing to make a Non Player Character (NPC) to appear according to an inference processing result, optimizes rendering processing, specifies an option selected based on the visual line information, and giving a notification for warning a bad health condition due to VR sickness of the equipment user U estimated based on the eyeball movement information or the pupil diameter information.

Furthermore, when implementing the function of the Cross Reality (XR) application, the application function unit F1 performs processing of changing a facial expression or a visual line of the avatar of the equipment user U according to the inference processing result, processing of making the avatar make a specific motion (gesture motion) in accordance with a feeling of the equipment user, and the like.

Furthermore, when implementing the function of the exercise application, the application function unit F1 performs processing of displaying a notification that recommends to lower an exercise strength according to the inference processing result, or the like.

Note that the control unit 3 comprehensively represents various types of arithmetic operation processing units including a Central Processing Unit (CPU), a Digital Signal Processor (DSP), and the like, and may include a plurality of arithmetic operation processing units.

The display unit 4 includes a display device that performs various types of display to the equipment user U. The display device is, for example, a display device such as a liquid crystal panel (LCD: Liquid Crystal Display) or an organic Electro-Luminescence (EL) display disposed in front of the equipment user U equipped with the HMD 1.

The display unit 4 executes various types of display on a display screen based on an instruction of the control unit 3. For example, the display unit 4 displays a Computer Graphics (CG) image as a display image of the VR space. Alternatively, the display unit 4 that performs display for an AR space superimposes an image of a virtual object in accordance with a position and a size of an object that exists in the real space to display.

Furthermore, the display unit 4 executes display of various types of User Interfaces (UIs) such as a menu screen based on an instruction of the control unit 3.

The storage unit 5 comprehensively represents a Read Only Memory (ROM), a Random Access Memory (RAM), and the like. Furthermore, the storage unit 5 may include a form of a memory card such as a flash memory.

The storage unit 5 stores various types of programs executed by the control unit 3, image data (image file) captured by the camera 7, meta data, and the like. Furthermore, the storage unit 5 stores the above-described imaging parameters.

The HMD 1 may include a communication unit and the like in addition to those in Fig. 2.

The communication unit can transmit and receive data by, for example, not only wired communication but also near field wireless communication.

### <2. Relationship between Acquired Information and Imaging Parameters>

Here, Fig. 10 illustrates an example of a relationship between the acquisition target information on the equipment user U, and the imaging parameters for suitably acquiring the acquisition target information.

First, the imaging parameters in a case where the acquisition target information is the "iris information" will be described.
Since a high-resolution image of the eyeball EB is suitable to obtain the iris information, the acquired image is the eyeball image.

Furthermore, it is suitable that the angle of the camera 7 is right in front of the eyeball EB.
As for the light sources 6 to be turned on, only the light sources 6 whose bright spots BP are located at portions other than the iris Ir are caused to emit light such that the bright spots BP do not exist on the iris Ir. Note that, since the light sources 6 whose bright spots BP are located on the iris Ir may change depending on a visual line direction of the equipment user U, it is preferable to turn off the light sources 6 associated with the bright spots BP on the iris Ir based on a captured image.

Note that, since the pupil Pu is preferably smaller to obtain the iris information, the pupil Pu of the equipment user U may be made smaller by displaying a high-brightness image on the display unit 4 located in front of the eyeball EB. Particularly, it is suitable to cause the light sources 6 to strongly emit light when the light sources 6 emit IR light because it is difficult to make the pupil Pu of the equipment user U smaller.
Furthermore, notification processing may be performed to ask the equipment user U refrain from blinking.

The light emission intensity of the light sources 6 that are caused to emit light is desirably as strong as possible as long as the eyes can be protected.

The wavelength of the light emitted from the light source 6 is preferably a wavelength close to the color of the iris Ir. Hence, the emission light from the light source 6 is preferably visible light rather than the IR light.

It is suitable that an imaging timing of the eyeball image for obtaining the iris information is a timing of equipment of the HMD 1, a time of purchase (at a time of payment) of a charging item, and the like. That is, the imaging timing is a timing to determine whether or not the equipment user U is a specific user.

Next, the imaging parameters in a case where the acquisition target information is "eyeball movement" or the "pupil diameter" will be described.
Since a high-resolution image of the eyeball EB is suitable to obtain information on the eyeball movement or the pupil diameter, the acquired image is the eyeball image.

Light sources to be turned on are preferably all of the light sources 6. Consequently, it is possible to acquire the eyeball image that is bright and suitable for image recognition processing.

It is suitable that the imaging timing of the eyeball image for obtaining the information on the eyeball movement or the pupil diameter is an information estimation timing, a timing to determine whether or not VR sickness occurs, a timing to determine a stress, and the like. By acquiring the eyeball movement, it is possible to estimate fluctuation information on autonomic nerves, so that it is possible to appropriately determine the stress.

Next, the imaging parameters in a case where the acquisition target information is the "visual line information" will be described.
Since a high-resolution image of the eyeball EB is suitable to obtain the visual line information, the acquired image is the eyeball image.

Furthermore, it is suitable that the angle of the camera 7 is right in front of the eyeball EB.

Since it is suitable that the multiple bright spots BP of the light sources to be turned on exist on the eyeball EB, it is preferable to turn on all of the light sources 6.

It is suitable that the imaging timing of the eyeball image for obtaining the visual line information is a UI operation timing, a timing during which foveated rendering is executed, a timing to estimate emotions, a timing to estimate a user experience, and the like. Consequently, it is possible to perform an operation matching the visual line information, preferentially improve a resolution of a virtual object located in the visual line information, and appropriately estimate the emotions or estimate the user experience.

Next, the imaging parameters in a case where the acquisition target information is the "facial expression information" will be described.
Since an image whose angle of view includes up to the surroundings of the eye is suitable to obtain the facial expression information, the acquired image is a wide angle image.

Furthermore, it is suitable that the angle of the camera 7 captures the eye and the surroundings of the eye from a diagonal direction.
As for the light sources to be turned on, for example, only the light sources 6 located below the center of the eyeball EB are caused to emit light to highlight ups and downs of the skin around the eye.

It is suitable that the imaging timing of the eyeball image for obtaining the facial expression information is a timing to estimate the emotions, a timing to estimate a user experience, and the like. Consequently, it is possible to appropriately estimate the emotions and estimate the user experience.

Next, the imaging parameters in a case where the acquisition target information is the "heart rate information" will be described.
Since an image whose angle of view includes up to the surroundings of the eye is suitable to obtain the heart rate information, the acquired image is a wide angle image.

The heart rate information can be acquired by capturing how the light amounts of reflection light of the light sources 6 periodically change due to pulsation of blood. Since this reflection light is weak, it is preferable to cause all of the light sources 6 to emit light. Furthermore, the light emission intensity of the light sources 6 that are caused to emit light is desirably as strong as possible as long as the eyes can be protected.

The wavelength of light emitted from the light source 6 is preferably a wavelength around 700 nm to 900 nm that is a wavelength whose light absorption rate of hemoglobins flowing in the blood is high. Consequently, it is possible to more greatly increase fluctuation of the reflection light due to pulsation.

It is suitable that the imaging timing of the eyeball image for obtaining the heart rate information is a timing to estimate the emotions, a timing to estimate a user experience, a timing to estimate an exercise amount, and the like. Consequently, it is possible to appropriately estimate the emotions, estimate the user experience, and estimate the exercise amount.

Note that it is also possible to estimate a respiratory rate of the equipment user U based on a change in the heart rate information. That is, a wide angle image may be captured using the imaging parameters used to obtain the heart rate information to obtain information on the respiratory rate of the equipment user U.

### <3. Processing Flow>

An example of processing executed by the control unit 3 of the HMD 1 will be described with reference to Fig. 11.

In step S101, the control unit 3 determines the acquisition target information according to the application. The application is implemented by the above-described application function unit F1, and is the user authentication application, the game application, and the like.

After determining the acquisition target information such as the visual line information or the iris information, the control unit 3 sets the imaging parameters matching the acquisition target information in step S102.

Subsequently, in step S103, the control unit 3 executes imaging matching the imaging parameters, and acquires image data.

In step S104, the acquired image data is input to the AI model to execute the inference processing. An inference result obtained here is used for various types of processing by the application function unit F1. For example, the user authentication application performs user authentication or identify authentication using the acquired iris information.

In step S105, the control unit 3 determines whether or not the application implemented by the application function unit F1 has been changed. It is conceived that, when, for example, authentication processing is executed using the iris information and an authentication result indicating a registered user is obtained, the application function unit F1 implements the function of the XR application as a next application.
In this case, in step S105, the control unit 3 determines that the application has been changed.

When determining that the application is not changed, the control unit 3 returns to processing in step S103, and continues imaging.

Note that, when determining that the application is not changed, the control unit 3 may return to step S102.
When, for example, the user authentication application functions, it is assumed that the eyeball EB of the user has moved and the visual line direction has changed. In this case, the light sources 6 to be turned off and the light sources 6 to be turned on may be preferably changed to obtain a suitable image of the iris Ir. In this case, the processing returns to processing in step S102 again to set the imaging parameters again and acquire image data in step S103.

When it is determined in step S105 that the application has been changed, the control unit 3 determines in step S106 whether or not to continue acquiring user information on the equipment user U. In, for example, the above-described example, it is determined to continue acquiring the user information such as diameter information of the pupil Pu or the heart rate information on the equipment user U to implement a desired function as the XR application.

When it is determined to continue acquiring the user information, the control unit 3 returns to processing in step S101, and determines the acquisition target information.

On the other hand, when it is determined not to continue acquiring the user information, the control unit 3 finishes a series of processing illustrated in Fig. 11.

The flowchart illustrated in Fig. 11 is an example, and Fig. 12 illustrates another example. Note that the same processing as that in Fig. 11 will be denoted by the same step numbers, and description thereof will be omitted as appropriate.
The example illustrated in Fig. 12 is an example where there are a plurality of types of the acquisition target information on the equipment user U. This example is, for example, a case where, while rendering processing is optimized by acquiring the visual line information on the equipment user U, the feeling of the equipment user U is reflected in the avatar by acquiring the facial expression information.

In step S101, the control unit 3 of the HMD 1 determines the acquisition target information according to the application. A plurality of pieces of the acquisition target information are determined here, and are, for example, the visual line information and the facial expression information.

In step S102, the control unit 3 sets the imaging parameters matching the acquisition target information. Here, for example, the imaging parameters for obtaining the visual line information are set first.

Subsequently, in step S103, the control unit 3 executes imaging matching the imaging parameters, and acquires image data, that is, the eyeball image.

In step S104, the acquired image data is input to the AI model to execute the inference processing. The visual line information that is the inference result obtained here is used for various types of processing by the application function unit F1.

In step S105, the control unit 3 determines whether or not the application implemented by the application function unit F1 has been changed.

When determining that the application is not changed, the control unit 3 determines in step S111 whether or not a switching timing has arrived. The switching timing is, for example, a timing to switch settings of the imaging parameters for obtaining the visual line information to settings of the imaging parameters for obtaining the facial expression information. The switching timing is determined according to the number of frame images captured after the imaging parameters are set, and the number of clocks that have passed.

When determining that the switching timing has not arrived, the control unit 3 returns to step S103, and continues imaging while the imaging parameters for obtaining the visual line information are set.

On the other hand, when determining that the switching timing has arrived, the control unit 3 returns to step S102, and sets the imaging parameters for obtaining the facial expression information.

Note that, since the same flow as that in Fig. 11 applies in a case where it is determined in step S105 that the application has been changed, description thereof will be omitted.

By executing a series of processing illustrated in Fig. 12, the control unit 3 can substantially simultaneously acquire a plurality of pieces of information that cannot be suitably acquired if the imaging parameters are not changed for the equipment user U.

### <4. Second Embodiment>

Fig. 13 illustrates a configuration of an HMD 1A according to a second embodiment. The HMD 1A according to the present embodiment includes the camera 7 for capturing an eyeball image, and the camera 7 for capturing a wide angle image. In this case, the above-described imaging parameters include information on which one of the cameras 7 to use for imaging.

The HMD 1A includes an eye tracker 2A, the control unit 3, the display unit 4, and the storage unit 5.
The eye tracker 2A includes the plurality of light sources 6 and cameras 7a and 7b.

The plurality of light sources 6 are disposed similarly to, for example, that illustrated in above-described Fig. 5.

The camera 7a of the two cameras 7a and 7b is provided to capture the eyeball image illustrated in Fig. 3.

Furthermore, the camera 7b is provided to capture the wide angle image illustrated in Fig. 4.

Fig. 14 illustrates an arrangement example of the light sources 6 and the cameras 7a and 7b. Note that Fig. 14 schematically illustrates only part of the plurality of light sources 6 disposed around the eye of the equipment user U.

The camera 7a is disposed at a position close to the eyeball EB, and thereby can capture the eyeball image.

Furthermore, the camera 7b is disposed at a position more distant from the eyeball EB than the camera 7a, and thereby can capture the wide angle image.

By providing the two cameras 7a and 7b, and switching a set of the camera 7 and the imaging parameters used for imaging, it is possible to alternately perform imaging performed by applying to the light sources 6 the imaging parameters for the camera 7a and imaging performed by applying to the light sources 6 the imaging parameters for the camera 7b.
Note that, when the parameters of the light sources 6 for the camera 7a and the parameters of the light sources 6 for the camera 7b are identical, it is possible to simultaneously obtain a plurality of pieces of information by causing the camera 7a and the camera 7b to simultaneously perform imaging.

Furthermore, although the camera 7a and the camera 7b illustrated in Fig. 14 are disposed at positions at which distances to the eyeball EB are different, a long distance in the visual line direction cannot be taken or when a much longer distance in the visual line direction needs to be taken, an aspect that a mirror M is used is also conceived.

More specifically, Fig. 15 illustrates this aspect.

The HMD 1A includes the plurality of light sources 6, two cameras 7c and 7d, and the mirror M.

The camera 7c is the camera 7 for acquiring the eyeball image, and is disposed at a position close to the eyeball EB.

The camera 7d is also disposed at a position close to the eyeball EB, yet performs imaging via the mirror M to image the wide angle image.

As described above, by performing imaging via the mirror M, it is possible to increase an imaging distance of the camera 7d, and capture an image of a wider angle of view.

Note that the HMD 1A includes one camera 7e, the camera 7e may substitute both functions of the camera 7a and camera 7b.

As illustrated in, for example, Fig. 16, the camera 7e may be movable between a first position Ps1 that is a position indicated by a solid line in Fig. 16 and is a position close to the eyeball EB, and a second position Ps2 that is a position indicated by a broken line in Fig. 16 and is a position distant from the eyeball EB.

When the camera 7e is located at the first position Ps1, the camera 7e can play a role of the camera 7a illustrated in Fig. 14 and, when the camera 7e is located at the second position Ps2, the camera 7e can play a role of the camera 7b illustrated in Fig. 14.

Consequently, it is possible to reduce the number of the cameras 7 and reduce cost.

Furthermore, when the two cameras 7a and 7b are substituted with the one camera 7, a configuration where the position of the camera 7 is not moved is also conceived.

For example, the HMD 1A may include one camera 7f as illustrated in Fig. 17. The camera 7f includes an imaging element unit 9 that includes a pixel array unit 8 inside, and an optical lens 10 on a light incidence side of the pixel array unit 8. The optical lens 10 is illustrated as one lens, yet may include a plurality of lenses.

The optical lens 10 is supported by a driving mechanism 11, and the optical lens 10 is configured to be drivable in an optical axis direction when a control signal S is applied to the driving mechanism 11 via the control unit 3 and a lens driver.

When the optical lens 10 is driven in the optical axis direction, it is possible to change the angle of view. That is, the camera 7f including the optical lens 10 that is movable in the optical axis direction can image both of the eyeball image and the wide angle image according to the position of the optical lens 10. In this case, the position of the optical lens 10 or the control signal S to be applied to the driving mechanism 11 is the imaging parameter.

### <5. Modification Example>

Part of information of the above described various pieces of information acquired for the equipment user U of the HMD 1 may be acquired from a device other than the HMD 1. For example, a device that can acquire the heart rate information may be attached to the neck or the wrist of the equipment user U, and the HMD 1 may communicate with the device to obtain the heart rate information on the equipment user U.

Consequently, it is possible to reduce a processing load of the image processing and a processing load of the inference processing of the HMD 1.

The camera 7 images the eyeball EB or the surroundings of the eye of the equipment user U in each of the above-described examples, yet may be disposed to image the mouth or a portion of the face other than the mouth of the equipment user U.

Consequently, it is possible to acquire the more detailed facial expression information or the like on the equipment user U.

Furthermore, the HMD 1 has a different distance to or positional relationship to the eyeball EB of the equipment user U every time the HMD 1 is equipped by the equipment user U. This difference may be caused by an individual difference of the equipment user U, yet may also occur even in a case of the same equipment user U.
To support the difference, the HMD 1 may make initial adjustment at a timing at which the equipment user U is equipped with the HMD 1. The initial adjustment is processing of adjusting the orientations and the positions of the light sources 6 and the cameras 7.
Consequently, the HMD 1 can capture the eyeball image and the wide angle image in an optimal state.

Furthermore, this adjustment may be executed not only at a timing at which the HMD 1 is equipped, but also every time a predetermined time passes or at a timing at which accuracy of the inference result on information obtained from the equipment user U lowers. In this case, the orientations and the positions of the light sources 6 and the cameras 7 are adjusted to increase the accuracy of the inference result. Furthermore, this adjustment may be executed by changing combinations of the orientations and the positions of the light sources 6 and the cameras 7 in a round robin way.

The light sources 6 that can emit light of multiple wavelengths may be used as the light sources 6, and multispectral cameras may be used as the cameras 7. In this case, by capturing images of the equipment user U using a plurality of wavelengths in an exploratory manner and comparing accuracy of information obtained by making an inference from the image, a wavelength range to use may be determined.

### <6. Summary>

As described in each of the above-described examples, the HMD 1 (1A) that is the head mount type information processing device includes the determination processing unit F2 that determines the acquisition target information according to a scene, the setting processing unit F3 that sets the imaging parameters for acquiring an image (e.g., an eyeball image or a wide angle image) that is an image of the eyes of the equipment user U and corresponds to the acquisition target information, and the imaging control unit F4 that captures the image of the eyes of the equipment user U according to the imaging parameters.

The scene described herein means an environment for specifying an application that needs to be executed, and is, for example, a scene in which the user authentication application is executed.

The acquisition target information differs depending on a use scene of the HMD 1 that is the head mount type information processing device, and is, for example, iris information, visual line information, facial expression information, heart rate information, or the like.

Suitable photographing conditions (imaging parameters) for obtaining these pieces of acquisition target information are different. Examples of the photographing conditions include a position and an angle of view of the camera 7, arrangement, a light amount, and a wavelength range of use of the light sources 6, and the like.

The acquisition target information is determined according to a scene, so that it is possible to appropriately set the imaging parameters.

Consequently, it is possible to capture appropriate images, and highly accurately obtain the acquisition target information such as the visual line information.

As described with reference to, for example, Figs. 3 and 4, an image of an eye may be an image (i.e., wide angle image) including a surrounding portion of the eye of the equipment user U.
By obtaining not only the image of the eyeball EB of the equipment user U, but also the image including the surrounding portion of the eye, it is possible to obtain information on wrinkles, the shape of the eyebrow, and the like around the eye. Consequently, it is possible to appropriately estimate the facial expression information of the user.

As described with reference to, for example, Fig. 10, in the HMD 1 that is the head mount type information processing device, the imaging parameters may be parameters of the camera 7.
Consequently, it is possible to acquire images in a state where the camera 7 is appropriately controlled according to the scene.

As described with reference to, for example, Fig. 13, the imaging control unit F4 of the HMD 1 that is the head mount type information processing device may select the camera 7 used for imaging from the plurality of cameras 7 according to the imaging parameters.
Consequently, when the plurality of cameras 7 whose angle of views or image sensors (imaging element units 9) are different types are prepared, it is possible to immediately switch the imaging parameters only by changing the camera 7. Consequently, it is possible to quickly obtain information that needs to be obtained according to a scene.

As described, the imaging control unit F4 of the HMD 1 that is the head mount type information processing device may control the position of the camera 7 according to the imaging parameters.
There may be, for example, a case where an appropriate position of the camera 7 differs depending on information that needs to be acquired. In this case, by changing the position of the camera 7 per information that needs to be acquired, it is possible to obtain appropriate images even when the plurality of cameras 7 are not provided.

As described with reference to, for example, Fig. 17, the imaging control unit F4 of the HMD 1 that is the head mount type information processing device may drive the optical lens 10 of the camera 7 used according to the imaging parameters.
There may be, for example, a case where an appropriate angle of view of the camera 7 differs depending on information that needs to be acquired. In this case, by driving the optical lens 10 per information that needs to be acquired, it is possible to change the angle of view of the camera 7. Consequently, it is not necessary to prepare the different camera 7 per information that needs to be acquired, and reduce cost.

As described with reference to, for example, Fig. 10, in the HMD 1 that is the head mount type information processing device, the imaging parameters may be parameters of the light sources 6.
Consequently, it is possible to acquire images in a state where the light sources 6 used for imaging are appropriately controlled according to the scene.

As described with reference to, for example, Figs. 6, 7, 8, 9, and 10, the imaging control unit F4 of the HMD 1 that is the head mount type information processing device may determine whether or not to cause the light sources 6 to emit light according to the imaging parameters.
When, for example, the plurality of light sources 6 for photographing are provided, it may be possible to capture images that need to be obtained by turning off part of the light sources 6. According to this configuration, it is possible to cause only the appropriate light sources 6 to emit light according to an image, and turn off the other light sources 6. Consequently, it is possible to capture appropriate images.

As described with reference to, for example, Fig. 10, the imaging control unit F4 of the HMD 1 that is the head mount type information processing device may determine the wavelength of light emitted from the light sources 6 according to the imaging parameters.
To obtain, for example, the iris information for iris authentication, it is preferable to image the iris Ir of the eyeball EB with a high resolution. Furthermore, by radiating from the light sources 6 the light of the appropriate wavelength adjusted to the color of the iris Ir, it is possible to obtain an image suitable for iris authentication. According to this configuration, the wavelength of the light emitted from the light sources 6 is determined according to the color of the iris Ir, so that it is possible to capture appropriate images for iris authentication.

As described above, the light sources 6 of the HMD 1 that is the head mount type information processing device emit infrared light (IR light).
By using light sources that use infrared light as the light source 6 and using an image sensor that has light reception sensitivity for the infrared light as the image sensor (imaging element unit 9), it is possible to capture images without dazzling the equipment user U. Consequently, it is possible to protect the eyes of the equipment user U, and prevent comfortableness of the equipment user U from being undermined.

As described with reference to, for example, Fig. 10, in the HMD 1 that is the head mount type information processing device, the acquisition target information may be information on the iris Ir of the equipment user U.
By obtaining information on the iris Ir from the image, the information can be used for iris authentication. Consequently, it is possible to perform identity authentication for user authentication or goods purchase in a state where the equipment user U is equipped with the HMD 1, and improve user-friendliness.

As described with reference to, for example, Fig. 10, in the HMD 1 that is the head mount type information processing device, the acquisition target information may be information on the visual lines of the equipment user U.
By obtaining the visual line information on the equipment user U from the image, it is possible to use the visual line information for a UI operation, and provide suitable operation means. Furthermore, by performing rendering processing based on the visual line information, it is possible to make the rendering processing efficient, and it is possible to reduce power consumption and increase the speed of the rendering processing.

As described with reference to, for example, Fig. 10, in the HMD 1 that is the head mount type information processing device, the acquisition target information may be information on the facial expression of the equipment user U. By obtaining the facial expression information on the equipment user U from the image, it is possible to reflect the facial expression information in the facial expression of the avatar of the equipment user U arranged in the virtual space, or make the avatar make a gesture corresponding to the facial expression information. Consequently, it is possible to make other users recognize the feeling corresponding to the facial expression of the equipment user U.

As described with reference to, for example, Fig. 10, in the HMD 1 that is the head mount type information processing device, the acquisition target information may be information on the heart rate of the equipment user U.
By obtaining information on the heart rate of the equipment user U from an image, it is possible to estimate a psychological state or the like of the equipment user U. Consequently, it is possible to launch a direction at a more effective timing in, for example, a horror game. Consequently, the equipment user U can obtain a more stimulating experience, and improve immersiveness.

As described with reference to, for example, Fig. 10, in the HMD 1 that is the head mount type information processing device, the acquisition target information may be information on the pupil diameter of the equipment user U. Furthermore, the acquisition target information may be information on the eyeball movement of the equipment user U.
By obtaining information on the pupil diameter or the eyeball movement of the equipment user U from an image, it is possible to estimate the emotions of the equipment user U. Consequently, it is possible to estimate a stress or the like based on VR sickness or a game experience, and make an appropriate instruction to the user.

As described above, the setting processing unit F3 of the HMD 1 that is the head mount type information processing device can switch between the first imaging parameter and the second imaging parameter as the imaging parameters to set, and the imaging control unit F4 may synchronize the camera clock signal used for imaging control of the camera 7 and the light source clock signal used for light emission control of the light sources 6, and switch between first imaging based on the first imaging parameter and second imaging based on the second imaging parameter at a timing matching frame imaging of the camera 7.
Consequently, for example, it is possible to switch between the first imaging and the second imaging per frame. Consequently, it is possible to substantially simultaneously acquire a plurality of pieces of information on the equipment user U, and it is possible to perform appropriate estimation processing based on the plurality of pieces of information on the equipment user U.

As described above, the HMD 1 that is the head mount type information processing device further includes the application function unit F1 for implementing a predetermined function, and the determination processing unit F2 may use a scene determined according to the predetermined function to determine the acquisition target information.
That is, the scene includes information indicating an application that needs to be executed. For example, in the scene in which the user authentication application is executed, the determination processing unit F2 determines the iris information on the equipment user U as the acquisition target information.
By determining a scene according to a specific function in this way, and determining the acquisition target information according to the scene, it is possible to acquire appropriate information per scene, and adjust the imaging conditions of the camera 7.

An information processing method according to the present technology performs processing of, at a computer device, determining the acquisition target information according to a scene, setting the imaging parameters for acquiring an image that is an image of the eyes of the equipment user U and corresponds to the acquisition target information, and capturing the image of the eyes of the equipment user U according to the imaging parameters.

A storage medium according to the present technology is a computer device-readable storage medium having stored thereon a program that causes an arithmetic operation processing to execute a function of determining the acquisition target information according to a scene, a function of setting the imaging parameters for acquiring an image that is an image of the eyes of the equipment user U and corresponds to the acquisition target information, and a function of capturing the image of the eyes of the equipment user U according to the imaging parameters.

A program according to the present technology is a program that causes, for example, an arithmetic operation processing device (control unit 3) such as the CPU included in the HMD 1 to execute a function of determining the acquisition target information according to a scene; a function of setting the imaging parameters for acquiring an image that is an image of the eyes of the equipment user U and corresponds to the acquisition target information; and a function of capturing the image of the eyes of the equipment user U according to the imaging parameters.
Such a program can implement various types of processing for obtaining information on the above-described equipment user U by the HMD 1 that is the above-described head mount type information processing device.

These programs can be stored in advance in a Hard Disk Drive (HDD) that is a recording medium built in a device such as a computer device, a ROM in a microcomputer including a CPU, or the like. Alternatively, the programs can be stored (recorded) temporarily or perpetually on a removable recording medium such as a flexible disc, a Compact Disk Read Only Memory (CD-ROM), a Magneto Optical (MO) disc, a Digital Versatile Disc (DVD), a Blu-ray Disc (registered trademark), a magnetic disk, a semiconductor memory, or a memory card. The removable recording medium can be provided as so-called package software. Furthermore, the programs can be installed from the removable recording medium to a personal computer or the like and can also be downloaded from a download site via a network such as a Local Area Network (LAN) or the Internet.

Note that the advantageous effects described in the present specification are merely exemplary and are not limited, and other advantageous effects may be obtained.

Moreover, the above-mentioned examples may be combined in any way, and even when various combinations are used, it is possible to obtain the various effects described above.

### <7. Present technique>

The present technique can also be configured as follows.
(1) A head mount type information processing device includes:
   a determination processing unit that determines acquisition target information according to a scene;
   a setting processing unit that sets an imaging parameter for acquiring an image that is an image of eyes of an equipment user and corresponds to the acquisition target information; and
   an imaging control unit that captures the image of the eyes of the equipment user according to the imaging parameter.
(2) In the head mount type information processing device described in above (1), the image of the eyes is an image including a surrounding portion of the eyes of the equipment user.
(3) In the head mount type information processing device described in any one of above (1) and above (2), the imaging parameter is a parameter of a camera.
(4) In the head mount type information processing device described in above (3), the imaging control unit selects a camera used for imaging from a plurality of cameras according to the imaging parameter.
(5) In the head mount type information processing device described in any one of above (3) and above (4), the imaging control unit controls a position of the camera according to the imaging parameter.
(6) In the head mount type information processing device described in any one of above (3) to above (5), the imaging control unit drives an optical lens included in the camera according to the imaging parameter.
(7) In the head mount type information processing device described in any one of above (1) to above (6), the imaging parameter is a parameter of a light source used for imaging.
(8) In the head mount type information processing device described in above (7), the imaging control unit determines whether or not to cause the light source to emit light according to the imaging parameter.
(9) In the head mount type information processing device described in any one of above (7) and above (8), the imaging control unit determines a wavelength of light emitted from the light source according to the imaging parameter.
(10) In the head mount type information processing device described in any one of above (7) to above (9), the light source emits infrared light.
(11) In the head mount type information processing device described in any one of above (1) to above (10), the acquisition target information is information on an iris of the equipment user.
(12) In the head mount type information processing device described in any one of above (1) to above (11), the acquisition target information is information on a visual line of the equipment user.
(13) In the head mount type information processing device described in any one of above (1) to above (12), the acquisition target information is information on a facial expression of the equipment user.
(14) In the head mount type information processing device described in any one of above (1) to above (13), the acquisition target information is information on a heart rate of the equipment user.
(15) In the head mount type information processing device described in any one of above (1) to above (14), the acquisition target information is information on a pupil diameter of the equipment user.
(16) In the head mount type information processing device described in any one of above (1) to above (15), the acquisition target information is information on eyeball movement of the equipment user.
(17) In the head mount type information processing device described in any one of above (1) to above (16),
   the setting processing unit is able to switch between a first imaging parameter and a second imaging parameter to set as the imaging parameter, and the imaging control unit
   synchronizes a camera clock signal used for imaging control of a camera, and a light source clock signal used for light emission control of a light source, and switches between first imaging based on the first imaging parameter and second imaging based on the second imaging parameter at a timing matching frame imaging of the camera.
(18) The head mount type information processing device described in any one of above (1) to above (17) further includes an application function unit for implementing a predetermined function, and
   the scene is determined according to the predetermined function.
(19) An information processing method causing a computer device to execute processing of:
   determining acquisition target information according to a scene;
   setting an imaging parameter for acquiring an image that is an image of eyes of an equipment user and corresponds to the acquisition target information; and capturing the image of the eyes of the equipment user according to the imaging parameter.
(20) A computer device-readable storage medium having stored thereon a program that causes an arithmetic operation processing device to execute:
   a function of determining acquisition target information according to a scene;
   a function of setting an imaging parameter for acquiring an image that is an image of eyes of an equipment user and corresponds to the acquisition target information; and
   a function of capturing the image of the eyes of the equipment user according to the imaging parameter.

### [Reference Signs List]

- 1, 1A: HMD (head mount type information processing device)
- 6, 6a, 6b, 6c, 6d, 6e, 6f, 6g, 6h: Light source
- 7, 7a, 7b, 7c: Camera
- 10: Optical lens
- U: Equipment user
- F2: Determination processing unit
- F3: Setting processing unit
- F4: Imaging control unit
- Ir: Iris

## Claims

1. A head mount type information processing device comprising:
a determination processing unit (F2) that determines acquisition target information according to a scene;
a setting processing unit (F3) that sets an imaging parameter for acquiring an image that is an image of eyes of an equipment user (U) and corresponds to the acquisition target information; and
an imaging control unit (F4) that captures the image of the eyes of the equipment user according to the imaging parameter,
**characterised in that**:
the imaging parameter is a parameter of a camera (7) and the imaging control unit is configured to:
select a camera used for imaging from a plurality of cameras according to the imaging parameter;
control a position of the camera according to the imaging parameter; or
drive an optical lens included in the camera according to the imaging parameter; and/or
the imaging parameter is a parameter of a light source (6) used for imaging and the imaging control unit is configured to:
determine whether or not to cause the light source to emit light according to the imaging parameter; or
determine a wavelength of light emitted from the light source
according to the imaging parameter.

2. The head mount type information processing device according to claim 1, wherein
the image of the eyes is an image including a surrounding portion of the eyes of the equipment user.

3. The head mount type information processing device according to claim 1, wherein
the acquisition target information is information on an iris or pupil diameter of the equipment user.

4. The head mount type information processing device according to claim 1, wherein
the acquisition target information is information on a visual line of the equipment user.

5. The head mount type information processing device according to claim 1, wherein the acquisition target information is information on a facial expression of the equipment user.

6. The head mount type information processing device according to claim 1, wherein the acquisition target information is information on a heart rate of the equipment user.

7. The head mount type information processing device according to claim 1, wherein the acquisition target information is information on eyeball movement of the equipment user.

8. The head mount type information processing device according to claim 1, wherein
the setting processing unit is able to switch between a first imaging parameter and a second imaging parameter to set as the imaging parameter, and
the imaging control unit
synchronizes a camera clock signal used for imaging control of a camera, and a light source clock signal used for light emission control of a light source, and
switches between first imaging based on the first imaging parameter and second imaging based on the second imaging parameter at a timing matching frame imaging of the camera.

9. The head mount type information processing device according to claim 1, further comprising an application function unit (F1) for implementing a predetermined function,
wherein the scene is determined according to the predetermined function.

10. An information processing method causing a computer device to execute processing of:
determining acquisition target information according to a scene;
setting an imaging parameter for acquiring an image that is an image of eyes of an equipment user and corresponds to the acquisition target information; and
capturing, by an imaging control unit, the image of the eyes of the equipment user according to the imaging parameter,
**characterised in that**:
the imaging parameter is a parameter of a camera and the imaging control unit is configured to:
select a camera used for imaging from a plurality of cameras according to the imaging parameter;
control a position of the camera according to the imaging parameter; and/or
drive an optical lens included in the camera according to the imaging parameter; and/or
the imaging parameter is a parameter of a light source used for imaging and the imaging control unit is configured to:
determine whether or not to cause the light source to emit light according to the imaging parameter; or
determine a wavelength of light emitted from the light source according to the imaging parameter.

11. A non-transitory machine-readable storage medium which stores computer software comprising instructions which, when the software is executed by a computer, causes the computer to carry out the method of claim 10.

## Patentansprüche

1. Kopfmontierbare Informationsverarbeitungsvorrichtung, umfassend:
eine Bestimmungsverarbeitungseinheit (F2), die Erfassungszielinformationen gemäß einer Szene bestimmt;
eine Einstellverarbeitungseinheit (F3), die einen Bildgebungsparameter zum Erfassen eines Bildes einstellt, das ein Bild von Augen eines Ausrüstungsbenutzers (U) ist und den Erfassungszielinformationen entspricht; und
eine Bildgebungssteuereinheit (F4), die das Bild der Augen des Ausrüstungsbenutzers gemäß dem Bildgebungsparameter aufnimmt,
**dadurch gekennzeichnet, dass:**
der Bildgebungsparameter ein Parameter einer Kamera (7) ist und die Bildgebungssteuereinheit konfiguriert ist zum:
Auswählen einer für die Bildgebung verwendeten Kamera aus einer Vielzahl von Kameras gemäß dem Bildgebungsparameter;
Steuern einer Position der Kamera gemäß dem Bildgebungsparameter; oder
Ansteuern einer in der Kamera eingeschlossenen optischen Linse gemäß dem Bildgebungsparameter; und/oder
der Bildgebungsparameter ein Parameter einer für die Bildgebung verwendeten Lichtquelle (6) ist und die Bildgebungssteuereinheit konfiguriert ist zum:
Bestimmen, ob ein Abgeben von Licht durch die Lichtquelle gemäß dem Bildgebungsparameter bewirkt werden soll oder nicht; oder
Bestimmen einer Wellenlänge von Licht, das von der Lichtquelle emittiert wird, gemäß dem Bildgebungsparameter.

2. Informationsverarbeitungsvorrichtung vom kopfmontierbaren Typ nach Anspruch 1, wobei das Bild der Augen ein Bild ist, das einen Umgebungsabschnitt der Augen des Ausrüstungsbenutzers einschließt.

3. Informationsverarbeitungsvorrichtung vom kopfmontierbaren Typ nach Anspruch 1, wobei die Erfassungszielinformationen Informationen über einen Iris- oder Pupillendurchmesser des Ausrüstungsbenutzers sind.

4. Kopfmontierbare Informationsverarbeitungsvorrichtung nach Anspruch 1, wobei die Erfassungszielinformationen Informationen über eine Sehlinie des Ausrüstungsbenutzers sind.

5. Informationsverarbeitungsvorrichtung vom kopfmontierbaren Typ nach Anspruch 1, wobei die Erfassungszielinformationen Informationen über einen Gesichtsausdruck des Ausrüstungsbenutzers sind.

6. Kopfmontierbare Informationsverarbeitungsvorrichtung nach Anspruch 1, wobei die Erfassungszielinformationen Informationen über eine Herzfrequenz des Ausrüstungsbenutzers sind.

7. Informationsverarbeitungsvorrichtung vom kopfmontierbaren Typ nach Anspruch 1, wobei die Erfassungszielinformationen Informationen über die Augenbewegungen des Ausrüstungsbenutzers sind.

8. Kopfmontierbare Informationsverarbeitungsvorrichtung nach Anspruch 1, wobei die Einstellungsverarbeitungseinheit in der Lage ist, zwischen einem ersten Bildgebungsparameter und einem zweiten Bildgebungsparameter umzuschalten, um diesen als den Bildgebungsparameter einzustellen, und die Bildgebungssteuerungseinheit
ein Kamerataktsignal synchronisiert, das für die Bildgebungssteuerung einer Kamera verwendet wird, und ein Lichtquellentaktsignal, das für die Lichtemissionssteuerung einer Lichtquelle verwendet wird, und zwischen einer ersten Bildgebung basierend auf dem ersten Bildgebungsparameter und einer zweiten Bildgebung basierend auf dem zweiten Bildgebungsparameter zu einem Zeitpunkt umschaltet, der mit der Bildframe-Erfassung der Kamera übereinstimmt.

9. Informationsverarbeitungsvorrichtung vom kopfmontierbaren Typ nach Anspruch 1, ferner umfassend eine Anwendungsfunktionseinheit (F1) zum Implementieren einer vorbestimmten Funktion,
wobei die Szene gemäß der vorbestimmten Funktion bestimmt wird.

10. Informationsverarbeitungseinrichtung zum Veranlassen einer Computervorrichtung, ein Verarbeiten auszuführen von:
Bestimmen von Erfassungszielinformationen gemäß einer Szene;
Einstellen eines Bildgebungsparameters zum Erfassen eines Bildes, das ein Bild von Augen eines Ausrüstungsbenutzers ist und den Erfassungszielinformationen entspricht; und Erfassen, durch eine Bildgebungssteuereinheit, des Bildes der Augen des Ausrüstungsbenutzers gemäß dem Bildgebungsparameter, **dadurch gekennzeichnet, dass:**
der Bildgebungsparameter ein Parameter einer Kamera ist und die Bildgebungssteuereinheit konfiguriert ist zum:
Auswählen einer für die Bildgebung verwendeten Kamera aus einer Vielzahl von Kameras gemäß dem Bildgebungsparameter;
Steuern einer Position der Kamera gemäß dem Bildgebungsparameter; und/oder
Ansteuern einer in der Kamera eingeschlossenen optischen Linse gemäß dem Bildgebungsparameter; und/oder
der Bildgebungsparameter ein Parameter einer für die Bildgebung verwendeten Lichtquelle ist und die Bildgebungssteuereinheit konfiguriert ist zum:
Bestimmen, ob ein Abgeben von Licht durch die Lichtquelle gemäß dem Bildgebungsparameter bewirkt werden soll oder nicht; oder
Bestimmen einer Wellenlänge von Licht, das von der Lichtquelle emittiert wird, gemäß dem Bildgebungsparameter.

11. Nicht flüchtiges maschinenlesbares Speichermedium, das Computersoftware speichert, Anweisungen umfassend, die, wenn die Software von einem Computer ausgeführt wird, den Computer veranlassen, das Verfahren nach Anspruch 10 durchzuführen.

## Revendications

1. Dispositif de traitement d'informations de type monture de tête comprenant :
une unité de traitement de détermination (F2) qui détermine des informations de cible d'acquisition selon une scène ;
une unité de traitement de définition (F3) qui définit un paramètre d'imagerie permettant d'acquérir une image qui est une image des yeux d'un utilisateur d'équipement (U) et correspond aux informations de cible d'acquisition ; et
une unité de commande d'imagerie (F4) qui capture l'image des yeux de l'utilisateur d'équipement selon le paramètre d'imagerie, **caractérisé en ce que** :
le paramètre d'imagerie est un paramètre d'une caméra (7) et l'unité de commande d'imagerie est configurée pour :
sélectionner une caméra utilisée pour l'imagerie à partir d'une pluralité de caméras selon le paramètre d'imagerie ;
commander une position de la caméra selon le paramètre d'imagerie ; ou
entraîner une lentille optique incluse dans la caméra selon le paramètre d'imagerie ; et/ou
le paramètre d'imagerie est un paramètre d'une source de lumière (6) utilisée pour l'imagerie, et l'unité de commande d'imagerie est configurée pour :
déterminer s'il faut ou non amener la source de lumière à émettre de la lumière selon le paramètre d'imagerie ; ou
déterminer une longueur d'onde de la lumière émise à partir de la source de lumière selon le paramètre d'imagerie.

2. Dispositif de traitement d'informations de type monture de tête selon la revendication 1, dans lequel l'image des yeux est une image comportant une partie environnante des yeux de l'utilisateur d'équipement.

3. Dispositif de traitement d'informations de type monture de tête selon la revendication 1, dans lequel les informations de cible d'acquisition sont des informations sur un diamètre de pupille ou d'iris de l'utilisateur d'équipement.

4. Dispositif de traitement d'informations de type monture de tête selon la revendication 1, dans lequel les informations de cible d'acquisition sont des informations sur une ligne visuelle de l'utilisateur d'équipement.

5. Dispositif de traitement d'informations de type monture de tête selon la revendication 1, dans lequel les informations de cible d'acquisition sont des informations sur une expression faciale de l'utilisateur d'équipement.

6. Dispositif de traitement d'informations de type monture de tête selon la revendication 1, dans lequel les informations de cible d'acquisition sont des informations sur une fréquence cardiaque de l'utilisateur d'équipement.

7. Dispositif de traitement d'informations de type monture de tête selon la revendication 1, dans lequel les informations de cible d'acquisition sont des informations sur un mouvement de globe oculaire de l'utilisateur d'équipement.

8. Dispositif de traitement d'informations de type monture de tête selon la revendication 1, dans lequel l'unité de traitement de définition est capable de commuter entre un premier paramètre d'imagerie et un second paramètre d'imagerie à définir comme paramètre d'imagerie, et l'unité de commande d'imagerie
synchronise un signal d'horloge de caméra utilisé pour la commande d'imagerie d'une caméra et un signal d'horloge de source de lumière utilisé pour la commande d'émission de lumière d'une source de lumière, et commute entre une première imagerie basée sur le premier paramètre d'imagerie et une seconde imagerie basée sur le second paramètre d'imagerie à un moment correspondant à l'imagerie de trame de la caméra.

9. Dispositif de traitement d'informations de type monture de tête selon la revendication 1, comprenant en outre une unité de fonction d'application (F1) pour mettre en oeuvre une fonction prédéterminée,
dans lequel la scène est déterminée selon la fonction prédéterminée.

10. Procédé de traitement d'informations amenant un dispositif informatique à exécuter un traitement consistant à :
déterminer des informations de cible d'acquisition selon une scène ;
définir un paramètre d'imagerie permettant d'acquérir une image qui est une image des yeux d'un utilisateur d'équipement et correspond aux informations de cible d'acquisition ; et capturer, par une unité de commande d'imagerie, l'image des yeux de l'utilisateur d'équipement selon le paramètre d'imagerie, **caractérisé en ce que :**
le paramètre d'imagerie est un paramètre d'une caméra et l'unité de commande d'imagerie est configurée pour :
sélectionner une caméra utilisée pour l'imagerie à partir d'une pluralité de caméras selon le paramètre d'imagerie ;
commander une position de la caméra selon le paramètre d'imagerie ; et/ou
entraîner une lentille optique incluse dans la caméra selon le paramètre d'imagerie ; et/ou
le paramètre d'imagerie est un paramètre d'une source de lumière utilisée pour l'imagerie et l'unité de commande d'imagerie est configurée pour :
déterminer s'il faut ou non amener la source de lumière à émettre de la lumière selon le paramètre d'imagerie ; ou
déterminer une longueur d'onde de la lumière émise à partir de la source de lumière selon le paramètre d'imagerie.

11. Support de stockage non transitoire lisible par machine qui stocke un logiciel informatique comprenant des instructions qui, lorsque le logiciel est exécuté par un ordinateur, amènent l'ordinateur à effectuer le procédé selon la revendication 10.
